# EUROPÄISCHE PATENTSCHRIFT

(11) **EP 2 701 599 B1**
(45) Veröffentlichungstag und Bekanntmachung des Hinweises auf die Patenterteilung: **27.05.2015**
(21) Anmeldenummer: 12719337.3
(22) Anmeldetag: 27.04.2012
(51) Int. Cl.: A61B 5/15, A61B 5/155, A61M 5/31, A61M 5/48

(54) **Fluidhandhabungsvorrichtung mit Federmechanismus**
Fluid handling device having a spring mechanism
Dispositif de manipulation de fluide équipé d'un mécanisme à ressort

(30) Priorität: 29.04.2011 DE 102011075028
(43) Veröffentlichungstag der Anmeldung: 05.03.2014
(73) Patentinhaber: Smiths Medical Deutschland GmbH, 85630 Grasbrun (DE)
(72) Erfinder: WEBER, Jörg, 83533 Edling (DE)
(74) Vertreter: Schenk, Markus
(86) Internationale Anmeldenummer: PCT/EP2012/057805
(87) Internationale Veröffentlichungsnummer: WO 2012/146735

(56) Entgegenhaltungen:
- EP-A2- 0 575 917
- WO-A1-01/24707
- WO-A1-97/18748
- DE-U1- 29 713 743
- US-A1- 2008 255 473

## Beschreibung

Die vorliegende Erfindung bezieht sich auf das technische Gebiet der Fluidhandhabung und insbesondere bezieht sich die vorliegende Erfindung auf eine Fluidhandhabungsvorrichtung, die als Blutentnahmevorrichtung in der Medizintechnik einsetzbar ist.

Für eine Tropfinfusion oder eine direkte Blutdruckmessung wird meist ein Infusionsschlauch oder ein Katheter in eine Vene oder Arterie eines Patienten eingesetzt, um beispielsweise für Untersuchungszwecke eine Blutprobe von einer außen zugänglichen Stelle des Infusionsschlauches zu entnehmen. Hierzu können beispielsweise Hähne oder bei neueren Systemen auch Entnahmestellen vorgesehen werden, die mit einem Innenlumen des Infusionssystems in Verbindung stehen und durch eine Kolbenspritze von außen zugänglich sind.

Bevor eine Blutentnahme aus diesem System vorgenommen werden kann, sollte zunächst dafür gesorgt werden, dass sich im Bereich der Entnahmestelle bzw. an dem Hahn nur unverdünntes Blut des Patienten und keine Anteile von Infusionslösungen oder Antikoagulationsmitteln befinden, die beispielsweise bei der direkten Blutdruckmessung zum Verhindern der Koagulation des Blutes zugeführt werden. Zu diesem Zweck sieht beispielsweise die WO 88/01846 A1 zwei Entnahmestellen vor, wobei die näher zum Patienten liegende Entnahmestelle der eigentlichen Entnahme der Blutprobe dient. Die weiter vom Patienten entfernt liegende hintere Entnahmestelle dient dazu, vorübergehend Infusionslösungen aus dem System zu entfernen, damit an der vorderen Entnahmestelle nur noch unverdünntes Blut vorhanden ist. Die WO 88/01846 A1 bietet somit eine verbesserte Möglichkeit zur Blutentnahme verglichen zu einer Vergehensweise nach der zunächst an einem Hahn mit einer Spritze Kochsalzlösung und Mischblut abgezogen wird, bis an dem Hahn reines Blut entnommen werden kann. Danach wird an diesem Hahn mit einer zweiten Spritze Blut zur Laboranalyse (beispielsweise zur Untersuchung von Blutgasen, etc.) entnommen. Die eingangs entnommene Menge aus Infusionslösung und Blut sollte dem Patienten nicht mehr zugeführt werden, da durch den Spritzenansaugvorgang Blutzellen geschädigt werden sowie eine Verunreinigung bzw. Kontamination des Blutes auftreten kann. Bei einer häufigen Blutentnahme führt dieses Abziehen von Mischblut vor jeder Blutentnahme ohne anschließende Rückführung zu einem spürbaren Flüssigkeitsverlust.

Bei der Ausführung nach der WO 88/01846 wird trotz der Kontaminationsgefahr und der Zellenschädigungen vorgeschlagen, die an der hinteren Entnahmestelle mittels einer herkömmlichen Spritze, deren Nadel einen ansonsten dichten Verschlussstopfen durchdringt, entnommene Menge dem System wieder zuzuführen. Dies setzt aber ein aufwändiges Hantieren voraus, und birgt neben der Gefahr, dass Verunreinigungen, Keime und sonstige Krankheitserreger dem System zugeführt werden, für das Klinikpersonal die Gefahr, dass es sich beim Entfernen der Nadel daran verletzt und infiziert, was in der Vergangenheit schon zu Aids- oder Hepatitisinfektionen beim Klinikpersonal geführt hat.

Es wurde daher schon vorgeschlagen, in soweit ein geschlossenes System zu verwenden, als die hintere Entnahmestelle als ein permanent in das Druckmess- oder Kathetersystem integrierter Zwischenspeicher mit einer Kolben-/Zylinder-Anordnung ausgestaltet ist, die von außen nicht mehr zugänglich ist, wie es beispielsweise in der Beschreibungseinleitung der EP 0575917 A2 berichtet wird. Bei dieser Blutentnahmevorrichtung tritt jedoch noch das Problem auf, dass der Zwischenspeicher bei Reinfusion des Patientenblutes nicht vollständig entleert wird, so dass dort verbleibende Blutreste koagulieren. Bei mehrmaliger Durchführung der oben genannten Vorgänge kann es somit vorkommen, dass koagulierte Blutreste aus dem Zwischenspeicher wieder zurück in den Blutkreislauf des Patienten gelangen und ihn hochgradig gefährdet.

Die EP 0575917A2 schlägt deshalb eine konusförmige Kolbenspitze in Verbindung mit einer konusförmlgen Zylinderraumspitze vor, wobei der Konuswinkel der Kolbenspitze größer ist als derjenige der Zylinderraumspitze, um beim Ausdrücken eine vollständige Entleerung zu gewährleisten. Es zeigt sich jedoch, dass selbst mit einer solchen Vorkehrung in einem geschlossenen Blutentnahmesystem, das über einen längeren Zeitraum (beispielsweise mehrere Tage) am Patienten angeschlossen ist, nicht sichergestellt werden kann, dass der Innenraum des Blutentnahmesystems, insbesondere des Zwischenspeichers, nicht durch Keimeinschleppung kontaminiert wird. Eine derartige Keimeinschleppung kann beispielsweise bei mehrmaligem Betätigen des Kolbens auftreten.

Ferner weisen herkömmliche Ansätze zur Blutentnahme mittels eines Zwischenspeichers den Nachteil auf, dass oftmals nicht sichergestellt werden kann, dass Blut- bzw. Infusionslösung aus dem Patienten bzw. aus dem Druckmessschlauch mit keinem zu großen Unterdruck herausgesaugt wird. Ein zu starker Unterdruck aufgrund beispielsweise eines zu starken Anziehens des Kolbens kann zur Folge haben, dass ein Ausgasen mit einer entsprechenden Bläschenbildung im Blut auftritt, und dass die Blutgefäßwand kollabiert bzw. ein Gefäßschluss resultiert, was je nach arterieller Entnahmestelle am Patienten zu Absterben von Gewebe bis hin zum To-de führen kann.

Eine zu starke Unterdruckbildung kann zwar durch ein entsprechend vorsichtiges Hantieren des Klinikpersonals beim Bedienen des Zwischenreservoirs verhindert werden, dies setzt aber einen entsprechend hohen Ausbildungs- und Wissensstand des Personals voraus. Unabhängig davon erfordert der Vorgang aufgrund der gebotenen Vorsicht einen hohen Zeitaufwand.

Schließlich erschwert ein Zwischenspeicher eines geschlossen Blutsystems die Sterilisation mittels beispielsweise ETO-Gas in einem Zustand, bei dem sich der Zwischenspeicher in einem zusammengebauten und beispielweise bereits in einem in einer gaspermeablen Verpackung angeordneten Zustand befindet.

Die DE 29713743 betrifft eine Vorrichtung zur Entnahme von Blut, bei der die Flüssigkeit nach der Entriegelung eines mit einer Kolbenstange verbundenen Griffes über die Kraft einer Feder automatisch und kontinuierlich in ein Gehäuse angesaugt wird, anschließend dieselbe Flüssigkeit wieder zurückgedrückt wird und der Griff über Vorsprünge eines Oberteiles arretiert wird, um in dieser Endstellung über einen an der Kolbenstange angeordneten Gummikolben mit einem an seiner Spitze befindlichen, vorzugsweise zylindrischen Vorsprung, der mit einem Radius versehen ist und über eine Bohrung im Gehäuse in den Flüssigkeitskanal der Vorrichtung hineinragt, eine vollständige Entleerung des Gehäuses zu gewährleisten.

Die US 2008255473 offenbart ein Fluidprobenahmesystem mit einer Fluidentnahmevorrichtung und einer Fluidprobenahmevorrichtung. Bei einer Ausführungsform weist die Fluidprobenahmevorrichtung einen Basisabschnitt mit einer Kanüle, einem Handgriff und einem Steg auf, der sich um zumindest einen Teil einer Außenoberfläche erstreckt; und einen oberen Abschnitt mit einem Flansch auf, der dazu angepasst ist, den Steg in Eingriff zu nehmen, um den oberen Abschnitt mit dem Basisabschnitt zu koppeln. Bei einer anderen Ausführungsform umfasst die Fluidprobenahmevorrichtung einen Basisabschnitt mit einer Kanüle, einem Griff, einem Anbringabschnitt und einem Teststreifen, der zumindest teilweise in dem Anbringabschnitt mit dem Basisabschnitt gekoppelt ist. Bei einer wieder anderen Ausführungsform umfasst die Fluidprobenahmevorrichtung ein Teststreifengehäuse zum Aufnehmen eines Endes eines Teststreifens darin. Eine stumpfe Kanüle erstreckt sich von einem Ende des Teststreifengehäuses.

Die Aufgabe der vorliegenden Erfindung besteht darin, eine Möglichkeit zu schaffen, dass ein Unterdruck bei einer Entnahme von Fluid, insbesondere Blut von einem Patienten, beschränkt werden kann, so dass insbesondere keine Gefäßwand eines Blutgefässes kollabiert oder keine Ausgasung des zu entnehmenden Fluids oder Blutes bewirkt wird.

Diese Aufgabe wird durch eine Fluidhandhabungsvorrichtung gemäß den Merkmalen des Patentanspruchs 1 gelöst.

Die vorliegende Erfindung schafft eine Fluidhandhabungsvorrichtung mit folgenden Merkmalen:
einem Aufnahmebehälter mit einem darin verschiebbar angeordneten Kolben, derart, dass durch eine Verschiebung des Kolbens das Volumen eines Fluidaufnahmereservoirs veränderbar ist;
einem Betätigungsmechanismus, der ausgebildet ist, um bei einer Betätigung desselben ein Mitnehmerlager zu verschieben; und
einem Federmechanismus, der ausgebildet ist, um eine Kraftübertragung von dem Mitnehmerlager auf den Kolben zu bewirken, um ansprechend auf eine Verschiebung des Mitnehmerlagers in eine erste Richtung eine solche Verschiebung des Kolbens in dem Aufnahmebehälter zu bewirken, dass ein Volumen des Fluidreservoirs erhöht wird.

Der vorliegenden Erfindung liegt die Erkenntnis zugrunde, dass durch die Entkopplung von dem Betätigungsmechanismus und der direkten Bewegung des Kolbens über den Federmechanismus nunmehr ein rasches Betätigen des Betätigungsmechanismus möglich ist, ohne dass ein zu starker Unterdruck in dem Entnahmesystem verursacht wird, durch den ansonsten beispielsweise die Gefäßwand eines Blutgefäßes bei dem Patienten kollabieren oder eine Ausgasung des von dem Patienten entnommenen Blutes stattfinden könnte. Vielmehr wird durch das Betätigen des Betätigungsmechanismus ein Federmechanismus gespannt, der durch eine langsame kontrollierte Abgabe der Spannungsenergie bewirkt, dass der Kolben nur mit einer mäßigen bzw. einer entsprechend vordefinierten Hubgeschwindigkeit angehoben wird, die z.B. zu einem begrenzten Unterdruck zum Rückziehen der Flüssigkeit und zur Entnahme des Blutes des Patienten führt.

Damit ergibt sich der Vorteil, dass eine Ausgasung einer in der an eine Öffnung des Reservoirs angrenzende Leitung befindlichen Flüssigkeit und des entnommenen Blutes vermieden wird und dass gegenüber herkömmlichen Ansätzen eine deutliche Abnahme der Gefahr eines Gefäßwandkollapses zu verzeichnen ist.

Der Federmechanismus kann dabei durch eine Schraubenfeder gebildet sein, was den Vorteil einer konstruktiv einfachen Lösung bietet, da eine Schraubenfeder ein einfaches und kostengünstiges mechanisches, Element darstellt, das leicht einbaubar ist.

Das Mitnehmerlager und/oder Kolben können derart ausgebildet sein, dass bei einer Verschiebung des Mitnehmerlagers in einer zweiten Richtung das Mitnehmerlager mit dem Kolben Eingriff nimmt, um eine solche Verschiebung des Kolbens zu bewirken, dass das Volumen des Fluidaufnahmereservoirs reduziert wird. Hierdurch kann vorteilhaft eine einfache Möglichkeit zu einer schnellen Entleerung des Aufnahmebehälter geschaffen werden, ohne konstruktiven Mehraufwand zu erfordern.

Der Kolben kann eine Kolbenstange aufweisen, die mit einer Kolbenscheibe versehen ist, wobei der Federmechanismus zwischen der Kolbenscheibe und dem Mitnehmerlager angeordnet ist. Dies bietet den Vorteil, durch eine derartige Anordnung eine konstruktiv einfache Lösung zum Spannen des Federmechanismus zu schaffen. Durch eine Verschiebung des Mitnehmerlagers in eine erste Richtung wird der Federmechanismus zwischen dem Mitnahmelager und der Kolbenstange gespannt.

Der Betätigungsmechanismus kann einen Drehkopf mit einem Gewindemechanismus aufweisen, wobei das Mitnehmerlager an einem Gewindeelement vorgesehen ist, das ein Gewinde aufweist, wobei der Gewindemechanismus in das Gewinde des Gewindeelementes unter gegenseitiger Verdrehsicherung eingreift, so dass durch eine Drehung des Drehkopfes die Verschiebung des Mitnehmerlagers bewirkt werden kann. Durch eine derartige Anordnung von Drehkopf, Mitnehmerlager und Gewindeelement kann vorteilhaft eine einfache Einstellung einer Position durch eine definierte Drehung am Drehkopf erreicht werden, was eine hochgenaue Einstellung eines Volumens des Fluidreservoirs ermöglicht.

Das Gewinde kann eine hohe Gewindesteigung aufweisen, derart, dass eine maximale Verschiebung des Mitnehmerlagers durch wenige, wie z.B. lediglich eine, Umdrehungen des Drehkopfes bewirkt werden kann. Hierdurch lässt sich vorteilhaft ein schnelles "Aufziehen" der Fluidhandhabungsvorrichtung durchführen, ohne dass ein Umgreifen notwendig ist.

Das Gewindeelement kann eine Innenbohrung aufweisen, an deren Ende das Mitnehmerlager gebildet ist, wobei der Kolben mit der Kolbenscheibe in das Ende der Innenbohrung hineinragt. Dies bietet den Vorteil, dass die Innenbohrung als Führung des Kolbens dienen kann.

Die Innenbohrung kann ausgebildet sein, um bei einer Verschiebung des Kolbens als eine Führung der Kolbenscheibe zu wirken, wobei der Federmechanismus in der Innenbohrung des Gewindeelementes zwischen dem Mitnehmerlager und der Kolbenscheibe angeordnet ist. In dieser Ausführungsform kann der Federmechanismus platzsparend in der Innenbohrung untergebracht werden, wodurch ein großes Volumen zur Fluidhandhabung zur Verfügung steht.

Die Innenbohrung kann eine Stufe aufweisen, die ausgebildet ist, um einen minimalen Abstand zwischen Kolbenscheibe und Mitnehmerlager sicherzustellen. Hierdurch wird ein Spannen des Federmechanismus über ein vorbestimmtes Maß hinaus verhindert und zudem ermöglicht, dass das Aufziehen des Aufnahmebehälters bzw. das Verschieben des Kolbens in die erste Richtung ab dem Zeitpunkt, da der Federmechanismus maximal gespannt ist bzw. die Stufe in die Kolbenscheibe eingreift, durch den Betreiber mittels einer durch den Betreiber auf den Betätigungsmechanismus ausgeübte Kraft unmittelbar bewerkstelligt werden kann, wodurch ein sicheres Ablösen eines Kolbenstopfens von einem mit der Behälteröffnung versehenen Behälterboden sichergestellt wird.

Der Kolben kann einen Kolbenstopfen aus einem reversibel verformbaren Material aufweisen, so dass durch eine Bewegung des Kolbenstopfens zu einem planaren Behälterboden Fluid aus der in dem planaren Behälterboden angeordneten Fluidöffnung treibbar ist, wobei der Kolbenstopfen in der dem planaren Behälterboden zugewandeten Oberfläche eine Ausnehmung aufweisen kann, derart, dass, wenn der Kolbenstopfen gegen den planaren Behälterboden gedrückt wird, bei zunehmendem Druck eine Berührungsgrenzlinie zwischen dem Kolbenstopfen und dem Behälterboden zu der Fluidöffnung wandert. Dies bietet den Vorteil, dass das Fluidreservoir beim Niederdrücken des Kolbens vollständig entleert werden kann. Alternativ kann der Kolben auch einen konischen Kolbenstopfen aufweisen.

Ein solcher konischer Kolbenstopfen kann ein reversibel verformbares Material aufweisen, wobei auch der Behälterboden eine konische Form aufweisen kann und wobei ein Konuswinkel des konischen Kolbenstopfens größer ist als ein Konuswinkel des konischen Behälterbodens. Dies bietet wieder den Vorteil, dass ein Fluidreservoir durch eine an der Konusspitze des Behälterbodens befindliche Fluidöffnung vollständig entleerbar ist.

Der Betätigungsmechanismus kann derart ausgebildet sein, um bei einer Bewegung des Kolbens in dem Aufnahmebehälter in eine erste Bewegungsrichtung den Federmechanismus zu spannen und bei einer Bewegung des Kolbens in einer zweiten Bewegungsrichtung, die der ersten Bewegungsrichtung entgegengesetzt ist, den Federmechanismus nicht zu spannen. Dies stellt sicher, dass der Federmechanismus bei einem Niederdrücken des Kolbens entspannt wird, wodurch sich die Lebensdauer eines solchen Federmechanismus erhöht.

Bevorzugte Ausführungsbeispiele werden anhand der beiliegenden Figuren näher beschrieben, wobei:
- Fig. 1A: bis 1D Seitenschnittansichten einer Fluidhandhabungsvorrichtung zu verschiedenen Zeitpunkten bei deren Betätigung gemäß einem Ausführungsbeispiel der vorliegenden Erfindung, wobei Fig. 1A lediglich eine Detailansicht darstellt;
- Fig. 1E: eine Teilschnittraumansicht des Kolbenstopfens von Fig. 1A-1D;
- Fig. 1F: und G eine Draufsicht bzw. eine Seitenansicht der Fluidhandhabungsvorrichtung von Fig. 1A-1D;
- Fig. 1H: eine Raumansicht der Fluidhandhabungsvorrichtung von Fig. 1A-1D mit entferntem Drehdeckel; und
- Fig. 2: eine Schnittansicht einer Fluidhandhabungsvorrichtung gemäß einem weiteren Ausführungsbeispiel der vorliegenden Erfindung in einer Stellung, die derjenigen von Fig. 1A entspricht.

In den nachfolgenden Zeichnungen werden gleiche oder ähnliche Elemente mit gleichen oder ähnlichen Bezugszeichen versehen, wobei eine wiederholte Beschreibung der Bezugszeichen weggelassen wird.

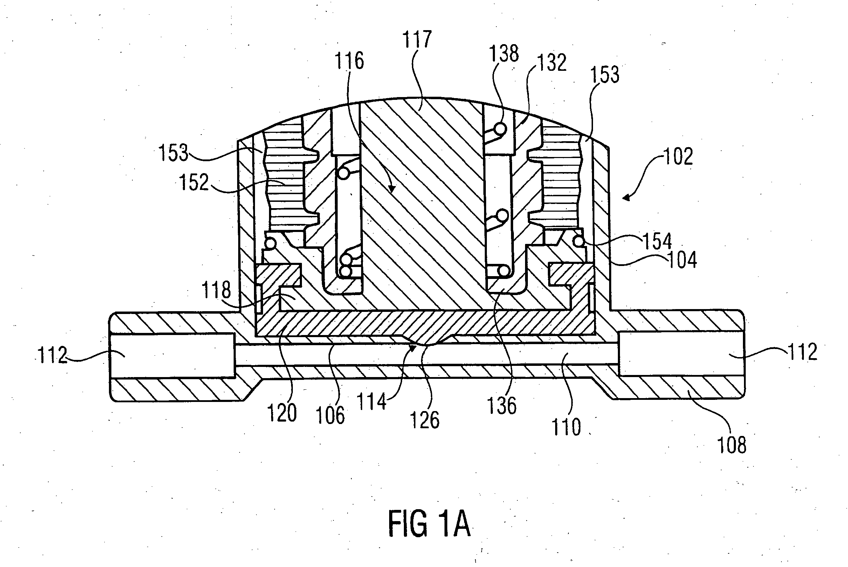

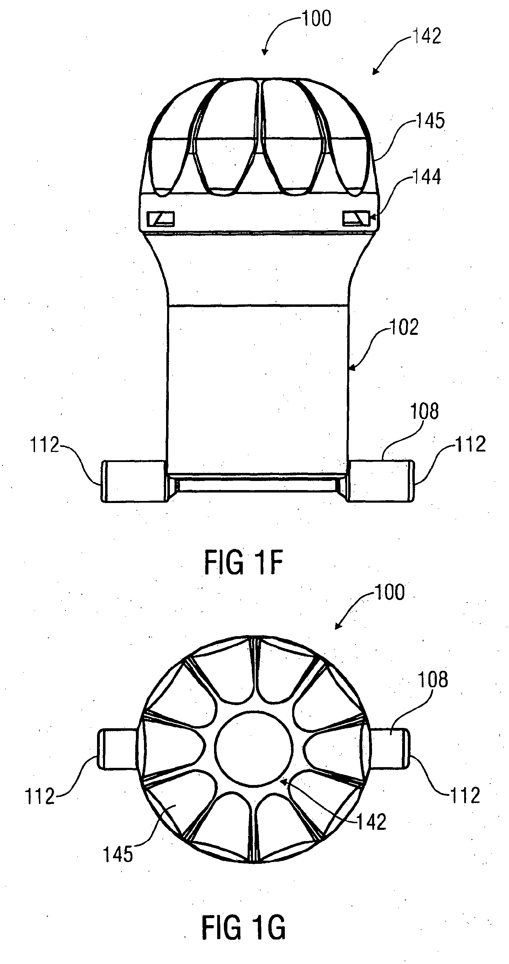

Die Fig. 1A zeigt eine Fluidhandhabungsvorrichtung 100 gemäß einem Ausführungsbeispiel der vorliegenden Erfindung. Hierbei weist die Fluidhandhabungsvorrichtung 100 einen Aufnahmebehälter 102 mit einer Seitenwand 104 sowie einen planaren (d.h. ebenen) Behälterboden 106 auf. Der Aufnahmebehälter 102 weist angrenzend an den Boden 106 einen ersten zylindrisch geformten Abschnitt 102a auf, an den sich wiederum ein zweiter zylindrisch geformter Abschnitt 102b mit einem größeren Innendurchmesser anschließt. Ferner ist der Aufnahmebehälter 102 an einem Verbindungsstück 108 befestigt, welches einen Durchflusskanal 110 zwischen zwei Anschlüssen 112 des Verbindungsstücks 108 aufweist. In dem ebenen Behälterboden 106 ist hier exemplarisch mittig eine Fluidöffnung 114 derart angeordnet, dass ein Fluidaustausch zwischen dem Verbindungskanal 110 und dem Inneren des Aufnahmebehälters 102 möglich ist.

Im Innern des Aufnahmebehälters 102 ist ein in Längsrichtung des Behälters 102 beweglicher Kolben 116 mit einer Kolbenstange 117 und einem an deren Ende angeordneten Kolbenstopfenträger 118 angeordnet, wobei der Kolbenstopfenträger 118 einen geringfügig kleineren Durchmesser als der Durchmesser des Abschnitts 102a aufweist und an dessen dem Boden 106 zugewandten Seite sowie dessen Umfangsseite ein elastisches, d.h. reversibel verformbares, Material als Kolbenstopfen 120 angebracht ist, so dass letzterer für ein fluiddichten Gleiten des Kolbens 116 entlang der Innenwand des Behälters 102 und damit einer Verdrängung des in dem Behälter 102 befindlichen Fluids, wie z.B. Infusionslösung bzw. Mischblut, aus dem Behälter 102 in den Kanal 110 bzw. ein Ansaugen von Fluid aus dem Kanal 110 sorgt.

In der dem Behälterboden 106 zugewandten Oberfläche des Kolbenstopfens, d.h. in dem elastischen. Material 120 desselben, ist eine hier exemplarisch ringförmige Ausnehmung 122 derart angeordnet, dass die Ausnehmung ausgehend von einem Seitenrand 124 derselben aus in Richtung der Fluidöffnung 114 zunächst eine zunehmende Ausnehmungstiefe aufweist, um sich im Bereich eines Verschlussstopfens 126 in dem elastischen Material 120, das in der Mitte des Kolbenstopfens angeordnet ist, wieder zu verringern, wobei der Verschlussstopfen 126 der Fluidöffnung 114 gegenüberliegend angeordnet ist. Anders ausgedrückt weist die Unterseite des Kolbenstopfens eine kreissymmetrische konkave Form auf. Andere Formen für die Ausnehmung sind allerdings ebenfalls möglich, wie z.B. eine mit (abschnittweise) konstanter und nicht kontinuierlich variierender Tiefe, und eine Ausnehmung ohne Verschlussstopfen 126 und beispielsweise monoton steigender Ausnehmungstiefe vom Rand bis zur Mitte.

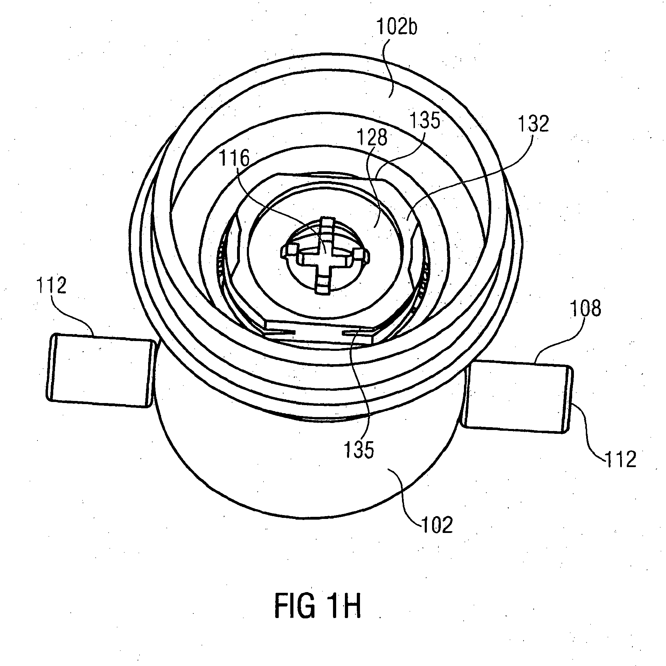

An einem oberen Ende des Kolbens 116 ist eine Kolbenscheibe 128 angeordnet bzw. an dem Kolben befestigt, die radial von der Kolbenstange 117 vorsteht und eine bewegliche Führung des Kolbens 116 in einer Bohrung 130 eines Gewindeelements 132 ermöglicht. Das Gewindeelement 132 und der Kolben 116 sind vorzugsweise derart in dem Aufnahmebehälter 102 angeordnet, dass eine relative Drehung zwischen Aufnahmebehälter 102 einerseits und dem Gewindeelement und wahlweise auch dem Kolben 116 andererseits nicht stattfindet. Hierzu weist das Gewindeelement 132 neben der Bohrung 130 eine im wesentlichen zylinderförmige Außenoberfläche auf, in der ein Außengewinde 134 und hier exemplarisch Abflachungen 135 gebildet sind, so dass sich das Gewindeelement 132 verdrehsichter durch eine entsprechend geformte Öffnung in einer Verdrehsicherungsscheibe 137 erstreckt, die wiederum selbst verdrehsicher bzw. gegen Verdrehung gehaltert auf an einer Grenze zwischen dem Abschnitt 102a und 102b in dem Behälter angeordnet ist. Die Abflachungen 135 sind in Fig. 1H zu erkennen. Ferner umfasst das Gewindeelement 132 an der unteren Seite einen als Mitnehmerlager fungierenden Flansch 136 auf, welcher nach innen, in Richtung des Kolbens 116 ragt, um eine Auflagefläche mit einem Loch zu bilden, durch das sich wiederum der Kolben 116 erstreckt.

Zwischen der Unterseite der Kolbenscheibe 128 und der Oberseite des Flansches 136 ist den Kolben 116 umgebend eine Schraubenfeder 138 angeordnet, die als Federmechanismus und hier insbesondere als Druckfeder wirkt. Ferner weist das Gewindeelement 132 in der Bohrung 130 einen Durchmesserwechsel auf, um an dem Flanschende breiter zu sein als an dem anderen Ende, wodurch in der Bohrung 130 eine ringförmige Auflage 140 gebildet wird. Der Außendruckmesser der Kolbenscheibe 128 ist so groß, dass sich die Kolbenscheibe 128 nur in dem weiter entfernt von dem Flansch 136 liegenden Teil der Bohrung 130 befinden kann, wodurch ein zu starkes Zusammendrücken der Schraubenfeder 138 verhindert wird, worauf im folgenden noch detaillierter eingegangen wird.

Ferner weist die Fluidhandhabungsvorrichtung 100 einen Drehdeckel 142 auf, der beispielsweise durch einen Schnappverschluss 144 an dem Aufnahmebehälter 102 befestigt ist, um die Öffnung des Behälters 102 von oben her in Form eines gewölbten Drehknopfes abzudecken und gegenüber dem Behälter 102 um eine gemeinsame Symmetrieachse von Behälter 102 und Drehdeckel 142 drehbar zu sein. Der Drehdeckel 142 umfasst an einer Innenseite eines gewölbten äußeren Griffteils 145 des Drehdeckels 142 einen von dieser Innenseite vorstehenden Gewindezylinder 146, der ein an dessen Innenseite gebildetes und in das Außengewinde 134 eingreifendes Innengewinde aufweist, wodurch mittels einer Drehung des Drehdeckels 142 eine Lage bzw. eine Höhe des verdrehsicher gelagerten Gewindeelements 132 in der Fluidhandhabungsvorrichtung 100 eingestellt werden kann.

Die Verbindung zwischen dem Drehdeckel 142 und dem Aufnahmebehälter 102 weist beispielsweise eine Belüftungsmöglichkeit in Form eines Spalts 148 zwischen dem Aufnahmebehälter 102 und dem Drehdeckel 142 auf, so dass ein Luftaustausch und damit ein Druckausgleich zwischen einem Umgebungsdruck außerhalb der Fluidhandhabungsvorrichtung 100 und einem Innendruck in der Fluidhandhabungsvorrichtung 100 möglich ist. Alternativ könnte eine gleichwirkende Öffnung in dem Drehdeckel 142 oder dem oberen Bereich des Behälters vorgesehen sein.

Durch eine relative Drehung zwischen Gewindemechanismus 132 und Drehdeckel 142 ist das Gewindeelement 132 in dem Gewindezylinder 146 ein- und ausfahrbar. Dazu ist, wie im vorhergehenden erwähnt, eine Verdrehsicherungsscheibe 137 vorgesehen, die ein Loch aufweist, durch welches sich das Gewindeelement 132 hindurcherstreckt, und das so geformt ist, dass sich das Gewindeelement 132 nicht gegenüber der Scheibe 137 drehen kann. Um die Verdrehsicherung gegenüber dem Behälter 102 zu komplettieren, ist auch die Scheibe 137 verdrehsicher bzw. -gehemmt in dem Behälter 102 angeordnet. Dazu ist ein oberer Dichtwulst 150 einer in Form einer im wesentlichen zylindrischen, ziehharmonika-artig geformten Membran gebildeten Dichtvorrichtung 152 zwischen eine Ringnut in einem ringförmigen Vorsprung zwischen dem Teil 102a mit geringerem Durchmesser und dem Teil 102b mit größerem Innendurchmesser des Behälters 102 und die Verdrehsicherungsscheibe 137 geklemmt, indem in dem zusammengebauten Zustand das Ende des vorstehenden Gewindezylinders 146 mit einer darin zur Zentrierung vorgesehenen Phase einen entsprechend geformten zylinderstumpfförmigen, Abschnitt der Scheibe 137 nach unten gegen den Dichtwulst 150 drückt. Der Dichtwulst 150 der Membran 152 sorgt durch seine Reibung nicht nur für eine Verdrehsicherung der Scheibe 137 gegenüber dem Behälter 102, sondern ferner für eine Dichtung, wie es im folgenden noch beschrieben wird.

Die Membran 152 erstreckt sich entlang der Behältersymmetrieachse von dem Übergang zwischen den Behälterabschnitten 102a und 102b aus bis zu dem Kolbenstopfenträger- 118 und umgibt dabei die Anordnung aus Kolben 116 und Gewindeelement 132, um - sich in Symmetrieachsenrichtung knautschend bzw. entfaltend - einen zwischen einem Teil der Innenwand 104 des Aufnahmebehälters 102, der sich zwischen dem Übergang zwischen den Behälterabschnitten 102a und 102b und der augenblicklichen Position des Kolbenstopfens 120 erstreckt, und der Membran 152 gebildeten Bereich 153 von dem übrigen, gegenüber Außen durch beispielsweise den Schlitz 148 belüfteten Innenraum des Aufnahmebehälters 102 keim- und luftundurchlässig zu trennen. Dazu ist die Membran 152 wie bereits erwähnt oben an dem Dichtwulst 150 abgedichtet befestigt. Zur Befestigung der Membran 152 an dem Kolbenstopfenträger 118 weist die Membran 152 auf dieser Seite beispielsweise ebenfalls einen Dichtwulst 154 auf, der umlaufend gebildet ist und unter leichter Spannung stehend in eine umlaufende Nut an einer Außenseite des Kolbenstopfenträgers 118 eingepasst ist. Die Membran besteht aus einem keim- und luftundurchlässigen Material, welches für infektiöses Material wie Bakterien, Viren oder Keime undurchlässig ist, wie z.B. Silikon.

Im oberen bzw. von dem Boden 106 entfernten Bereich des Abschnitts 102a des Aufnahmebehälters 102 ist in der Behälterwand 104 eine Öffnung 156 vorgesehen, durch welche unabhängig von der Stellung des Kolbens 116 ein Gasaustausch zwischen dem Bereich 153 und einer Außenumgebung der Fluidhandhabungsvorrichtung 100 ermöglicht wird. In dieser Öffnung 156 kann ein Filter 158 (z.B. ein Bakterienfilter) vorgesehen sein, um zu verhindern, dass Keime von der äußeren Umgebung in den Bereich 153 zwischen der Membran 152 und der Aufnahmebehälterinnenwand 104 eindringen und sich an der Gehäuseinnenwand 104 absetzen können, was bei Wiederausstoßen des zwischengespeicherten Fluids im Kolbenvolumen zu einer Kontamination des mit dem Kanal 110 verbundenen Blutkreislaufes führen könnte.

Im folgenden soll die Funktionsweise der erfindungsgemäßen Fluidhandhabungsvorrichtung 100 näher beschrieben werden. Hierbei soll zunächst von einem Grundzustand ausgegangen werden, in welchem das Gewindeelement 132 den niedrigst möglichen Zustand hat. Dieser Zustand ist in Fig. 1A dargestellt. In diesem Zustand ist das Gewindeelement 132 maximal aus dem Gewindezylinder 146 ausgedreht, um die dem Behälterboden 106 abgewandte Seite des Kolbenstopfenträgers 118 zu kontaktierten und hierüber den Kolbenstopfen 122 mit einer durch das Drehmoment an dem Drehdeckel 142 bestimmten Kraft gegen den Boden 106 zu pressen, so dass der Kolbenstopfen trotz Ausnehmung 122 in dem entspannten ungepressten Zustand plan an dem Boden 106 anliegt, jegliche Flüssigkeit aus dem Kolbeninneren in den Kanal 110 verdrängt worden ist und der Stopfen 126 mit der Innenwand des Kanals 110 bündig endet, so dass in dem Kanal kein Totzonenbereich mit der damit verbundenen Gefahr von Blut- und Keimanlagerungen entsteht. Die Feder 138 befindet sich in einem leicht vorgespannten Zustand, bei dem die Kolbenscheibe 128 gegen eine innerhalb der Bohrung 130 maximal von dem Flansch 136 entfernte Position gedrückt wird.

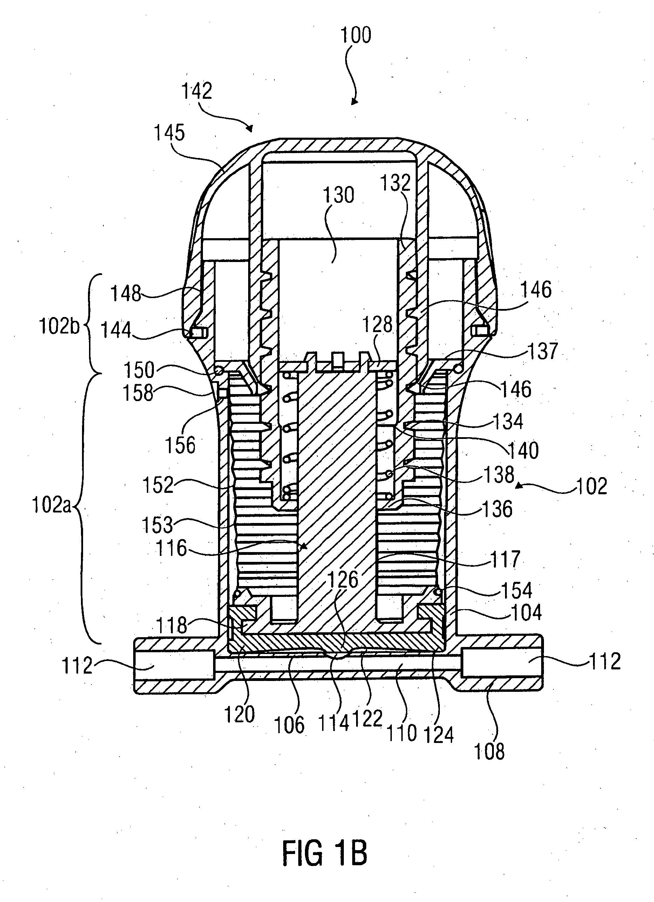

Wird nun eine Drehung des Drehdeckels 142 durchgeführt, wird das Gewindeelement 132 nach oben gezogen, wie es in Fig. 1B dargestellt ist. Hierdurch wird, verursacht durch das Anheben des Flansch 136, die Feder 138 zunächst weiter zusammengedrückt und gespannt, da der Kolben 116 mit der Kolbenscheibe 128 der Aufwärtsbewegung des Gewindeelements 132 zunächst nicht folgen kann bzw. nicht mit der gleichen Geschwindigkeit wie das Gewindeelement 132 angehoben wird, da sich zunächst der Kolbenstopfen 122 von dem Boden 106 ablösen muss. Die Entkopplung zwischen dem schnellen Anheben des Gewindeelements 132 und dem langsameren Anheben des Kolbens 116 durch die Schraubenfeder 138 hat den Vorteil, dass bei einer entsprechenden Ausgestaltung des Außengewindes 134 des Gewindeelements 132, insbesondere einer hohen Gewindesteigung, das Gewindeelement 132 durch wenige oder sogar mit nur einer Umdrehung bzw. durch einen geringen Drehweg sehr weit angehoben werden kann, ohne dass zugleich ein "Hochreißen" des Kolbens 116 resultiert, wodurch, wie vorstehend bereits ausgeführt, ein Ausgasen von Blut bzw. ein Kollaps einer Blutgefäßwand verhindert werden kann. Die Fig. 1B zeigt dabei einen Moment, bei dem der Drehdeckel 142 gerade etwas gedreht wurde, wobei das Gewindeelement 132 bereits deutlich angehoben wurde, während sich der Kolben 116 noch kaum bewegt hat.

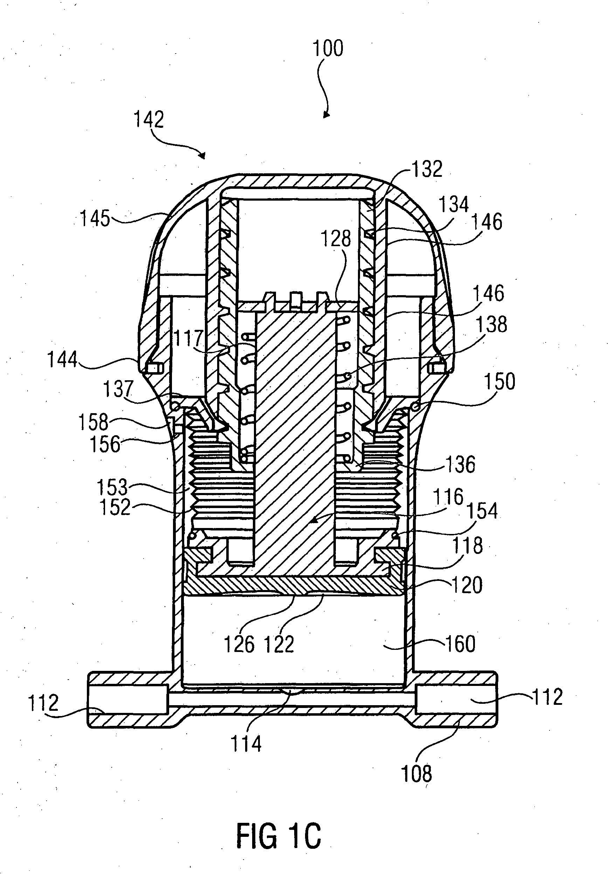

Die Fig. 1C stellt einen Zustand dar, bei der das Gewindeelement 132 bereits vollständig nach oben gedreht worden ist, der Kolbenstopfen 120 sich von dem Boden 106 gelöst hat und sich durch die Kraft der Schraubenfeder 138 der Kolben 116 mit dem Kolbenstopfen 120 nach oben bewegt, so dass Flüssigkeit aus dem Kanal 110 durch die Öffnung 114 in die Kolbenkammer angesaugt wird. Insbesondere hat sich in Fig, 1C der Kolbenstopfen 120 bereits um einen gewissen Weg nach oben bewegt, so dass im unteren Teil des Aufnahmebehälters 102 ein Fluid- bzw. Flüssigkeits/Mischblutreservoir 160 entstanden ist. Hierbei zeigt sich auch, dass die Membran 152 sich ziehharmonika-artig zusammenfalten kann, und somit einer Bewegung des Kolbens 116 folgt.

Bevor auf den weiteren Ansaugvorgang eingegangen wird, sei darauf hingewiesen, dass verschiedene Szenarien dafür existieren, wie weit die Feder 138 zwischen den Zuständen in den Fig. 1B und 1C zusammengedrückt wird. Es kann sein, dass angetrieben durch die Drehbewegung des Drehdeckels 142 sich das Gewindeelement 132 so schnell von dem Boden entfernt, dass sich der Kolben 116 noch immer in der Nähe der Ausgangsposition von Fig. 1A bzw. 1B befindet und deshalb die Auflage 140 die Kolbenscheibe 128 in Eingriff nimmt, so dass von da an die durch das an dem Drehdeckel 142 wirkende Drehmoment hervorgerufene, nach oben gerichtete Kraft auf das Gewindeelement 132 unmittelbar auch auf den Kolben 116 wirkt. Hierdurch wird ein sicheres Ablösen des Kolbenstopfens 120 von dem Behälterboden 106 sichergestellt, da die Ablösekraft nicht auf die maximale Kraft der Druckfeder 138 beschränkt ist, die durch den Abstand der Auflage 140 von dem Flansch 136 definiert ist. Andererseits ist die Auflage 140 an einer solchen Position angeordnet, dass der Verfahrweg des Gewindeelementes von der Stelle an, an der die Auflage 140 die Kolbenscheibe 128 frühestens kontaktieren kann (nämlich wenn sich der Kolben in der Position von Fig. 1A befindet), zu der Endposition, wie sie in Fig. 1C gezeigt ist, gering, so dass in der Zwischenzeit, in der sich ja der Kolben 116 mit der gleichen Geschwindigkeit wie das Gewindeelement 132 bewegt, keine Gefahr eines zu großen Unterdrucks in dem Kolbenvolumen und der damit verbundenen Blasenbildung besteht, und reicht gerade zum sicheren Ablösen des Stopfens 122.

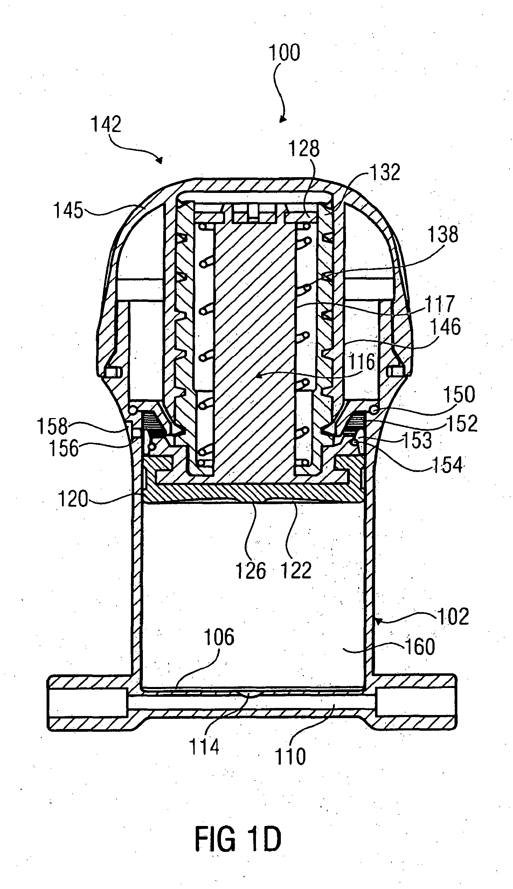

Die Fig. 1D zeigt die Fluidhandhabungsvorrichtung in einem Endzustand, das heißt, wenn sowohl das Gewindeelement 132 als auch der Kolben 116 in einer Position maximaler Höhe sind, so dass die Schraubenfeder 138 wieder entspannt (bzw. mit der vorbestimmten Vorspannung vorgespannt) ist. In diesem Fall ist auch die Membran 152 maximal zusammengefaltet, das heißt, ziehharmonika-artig im Bereich zwischen dem oberen Ende des Kolbenstopfens 118 und dem Gewindemechanismus 146 eingebettet. Das Blutreservoir 160 hat in dem in Fig. 1D dargestellten Zustand somit seine maximale Ausdehnung bzw. maximales Füllvolumen. In einem derartigen Zustand kann dann beispielsweise an einer zwischen dem Kanal 110 der Fluidhandhabungsvorrichtung und dem Patienten angeordneten Entnahmevorrichtung Blut entnommen werden, das nicht mit Infusionslösung vermischt ist, so dass somit keine Verfälschung der Analysewerte zu befürchten ist.

Um nun einen Flüssigkeitsverlust des Patienten durch die Blutentnahme möglichst gering zu halten, kann das bei der Blutabnahme in dem Blutreservoir 160 befindliche Blut anschließend wieder dem Patienten zugeführt werden. Dies kann dadurch erfolgen, dass der Drehdeckel 142 in die entgegengesetzte Richtung wie beim "Aufziehen" der Fluidhandhabungsvorrichtung gedreht wird, wodurch der Kolben 116 durch den unteren Teil des Flansch 136 bzw. des Gewindeelementes 132 niedergedrückt wird und dadurch ein Überdruck entsteht, aus dem ein Austreiben des in dem Blutreservoir 160 befindlichen Mischblutes durch die Fluidöffnung 114 in den Durchflusskanal 110 resultiert. Hierbei ist eine besondere Rücksichtnahme auf die Druckverhältnisse nicht derart kritisch, obwohl selbstverständlich ein Niederdrücken des Kolbens 136 durch ein Drehen am Gehäusedeckel 142 vom Klinikpersonal mit Vorsicht durchgeführt werden sollte. Einen Kollaps der Gefäßwand oder ein Ausgasen des Blutes kann jedoch in diesem Fall nicht auftreten.

Ist beim Austreiben durch das Gewindeelement 132 der Kolben 116 mit dem Kolbenstopfen 120 ausreichend niedergedrückt worden, so berührt aufgrund der Ausnehmung 122 zunächst ein Randbereich des reversibel verformbaren Materials 120 den ebenen Gefäßboden 106. Bei einem weiteren Niederdrücken des Kolbenstopfens 118 wird dann das elastische Material 120 derart verformt, dass sich die Ausnehmung 122 zunehmend schließt bzw. verkleinert, und zwar derart, dass eine die Fluidöffnung 114 umgebende Berührungsgrenzlinie zwischen dem elastischen Material 120 und dem ebenen Behälterboden 106 in Richtung der Fluidöffnung 114 wandert. Somit wird sichergestellt, dass das in dem Blutreservoir 160 vorhandene Blut vollständig aus dem Aufnahmebehälter 102 "herausgepresst" wird. Ferner kann, wenn das Blutreservoir 160 vollständig entleert ist, durch den Verschlussstopfen 126 die Fluidöffnung 114 vorzugsweise vollständig bündig mit der Kanalinnenwand verschlossen werden, wodurch sich eine Koagulation aufgrund eines Totzonenbereichs im Durchflusskanal 110 vermeiden lässt. Der Verschlussstopfen 126 ist wie im vorhergehenden beschrieben vorzugsweise an die Form der Fluidöffnung 114 angepasst sein, um bündig mit der Kanalinnenwand abzuschließen.

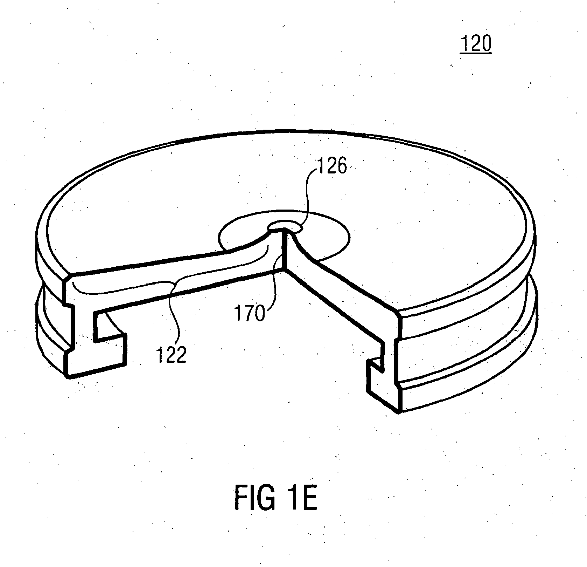

Gemäß einer Ausführungsform kann die Ausnehmung 122 in dem Kolbenstopfen 120 von einer Draufsicht auf den Stopfen 120 aus betrachtet einen kreisringförmige Form um ein Zentrum 170 herum haben, das durch die Fluidöffnung 114 bzw. die Symmetrieachse beispielsweise des Behälters 102 definiert ist, so wie es in der Fig. 1E dargestellt ist. Alternativ könnte die Ausnehmung 122 oval um die Mitte 170 herum angeordnet sein. Entsprechend kann auch die Fluidöffnung 114 kreisförmig oder schlitzförmig sein.

Wenn das Blut vollständig aus dem Aufnahmebehälter 102 herausgepresst ist, ist die Fluidhandhabungsvorrichtung wieder in einem Zustand, wie er in Fig. 1A dargestellt ist.

Durch die Öffnung 156 in der seitlichen Behälterwand 104 ist ein Gasaustausch zwischen dem durch die Membran 152 und die Behälterinnenwand 104 begrenzten Bereich 153 mit Außen möglich, nämlich eine Entlüftung des Raumes 153 bei Verfahren des Kolbens nach oben und eine Belüftung bei Verfahren des Kolbens nach unten, wobei in letztgenanntem Fall der Filter 158 Keime am Eindringen in den Raum 153 hindert. Der Filter 158 kann beispielsweise eine papierartige Membran sein, die entlang ihres Randes durch Haftmittel bzw. Kleber, eine Schweißnaht oder einen Halte- bzw. Klemmring an dem Gehäuse 102 in der Öffnung 120 befestigt ist.

Die Be- und Entlüftungsmöglichkeit über die Öffnung 156 bietet auch einen Vorteil bei einem Sterilisieren der Fluidhandhabungsvorrichtung 100, wie z.B. nach deren Zusammenbau und Einbringen in eine geeignete Verpackung, wie z.B. einer ETO-Gas-permeablen Verpackung, da sich der Raum 153 vorab evakuieren und dann mit dem ETO-Gas füllen lässt. Keime, die sich an der Behälterinnenwand 104 angelagert haben, können so durch die Sterilisation mit einem Sterilisationsgas, wie z.B. ETO, unschädlich gemacht werden. Durch den Bakterienfilter 158 kann dann auch verhindert werden, dass weitere Keime beim Einsatz der Fluidhandhabungsvorrichtung 100 in den Bereich 153 zwischen der Membran 152 und der Behälterinnenwand 104 eindringen können.

Gegenüber herkömmlichen Ansätzen bietet eine solche Anordnung der Öffnung 156, der Membran 152 und des Bakterienfilters 158 den Vorteil, dass nicht mehr ein großer Bakterienfilter, beispielsweise an der Oberseite des Drehdeckels, notwendig ist, durch den ein großvolumiger Luftaustausch und somit ein Druckausgleich beim Betätigen der Fluidhandhabungsvorrichtung ermöglicht wird. Vielmehr kann ein wesentlich kleinerer Bakterienfilter 158 verwendet werden, da eine deutlich geringere Menge von Gasvolumen auszutauschen ist.

Lediglich der Vorsicht halber wird darauf hingewiesen, dass außer zur Zwischenspeicherung von Blut die Fluidhandhabungsvorrichtung auch zur Handhabung von anderen Fluiden bzw. flüssigkeiten verwendet werden kann, wie sie beispielsweise in der Chemie und Biochemie verwendet werden, wobei sich hier ähnliche Vorteile ergeben können durch beispielsweise die oben beschriebene Vermeidung einer Blasenbildung, die vollständigen Entleerung der Kolbenkammer und die Sterilisierbarkeit des an die Kolbenkammerinnenseite angrenzenden Raums 153.

Zusammenfassend wurde im vorhergehenden eine Fluidhandhabungsvorrichtung beschrieben, die insbesondere als Blutentnahmereservoir Bestandteil eines geschlossenen Blutentnahmesystems sein kann. Über das Reservoir wird die Flüssigkeitssäule in einem mit dem Kanal 110 gekoppelten Schlauch zurückgezogen, bis an einer patientennäheren Entnahmestelle entlang des Schlauches beispielsweise Reinblut entnommen werden kann. Die Entnahme kann über einen Entnahmeadapter durch eine Punktionsmembrane erfolgen. Das zurückgezogene Flüssigkeitsvolumen im Reservoir kann anschließend dem Patienten wieder zugeführt werden, so dass ein signifikanter Flüssigkeitsverlust bei häufigen Blutproben bzw. -entnahmen vermieden werden kann. Da ein geschlossenes Blutentnahmesystem über einen längeren Zeitraum (beispielsweise mehrere Tage) am Patienten angeschlossen sein kann, muss sichergestellt sein, dass der Innenraum des Systems nicht durch Keimeinschleppung kontaminiert wird. Hierfür ist die vorerwähnte Membrane zwischen Gehäuse und Kolben angebracht. Während der Sterilisation, die in dem zusammengebauten bzw. verpackten Zustand stattfinden kann, kann durch die Öffnung 156 ETO-Gas als ein exemplarisches Sterilisationsgas (ETO = Ethylenoxid) an die Gehäuseinnenwand gelangen und anschließend wieder entfernt werden. Der Raum zwischen Gehäuse und Membrane ist aber dennoch auch während des Einsatzes steril, wofür der Bakterienfilter sorgt, der einen Gasaustausch ermöglicht, eine Migration von Keimen jedoch unterbindet. Durch die Feder wird sichergestellt, dass der Kolbenstopfen nur so schnell zurückgezogen wird, dass es zu keiner Entgasung in der Flüssigkeit bzw. keinem Kollabieren der Gefäßwände in Folge eines zu großen Unterdrucks kommt. Darüber hinaus ermöglicht diese Entkopplung eine optimierte Gewindesteigung zwischen Drehdeckel und Kolben, so dass mit wenigen Umdrehungen, wie z.B. lediglich einer, der Kolben bis zum Anschlag gezogen werden kann. Eine flache Steigung, um die Rückzugsgeschwindigkeit des Kolbens zu limitieren, ist nicht notwendig, so dass auch bei größeren Volumina die Handhabung praktikabel bleibt, da der Drehdeckel nicht so häufig gedreht werden muss.

Vorteilhaft ist die Fluidhandhabungsvorrichtung von Fig. 1A-H auch in herstellungstechnischer Hinsicht, da der Zusammenbau ein einfacher und schneller Prozess ist. Insbesondere wird beim Zusammenbau zunächst der Kolben 116 mit dem Kolbenstöpfenträger. 118 und dem daran befestigten elastischen Material 120 durch das Loch an dem Flanschende des Gewindeelementes 132 gesteckt und die Feder 138 in die Bohrung 130 eingefügt. Die Feder 138 leicht zusammendrückend wird dann die Kolbenscheibe 128 an dem dem Kolbenstopfenträger 118 gegenüberliegenden Ende des Kolbens 116 befestigt. Daraufhin wird die Membran 152 mittels des Dichtwulstes 154 an dem Kolbenstopfenträger 118 befestigt, und zwar hier exemplarisch mittels Schnappverschluss (Fig. 1A). Diese Anordnung wird dann in den Behälter 102 eingefügt, wobei der Dichtwulst 150 in dem Übergang zwischen den Abschnitten 102a und 102b des Behälters 102 angeordnet wird. Daraufhin wird die Verdrehsicherungsscheibe in den Abschnitt 102b eingefügt, um auf dem Dichtwulst 150 aufzuliegen. Schließlich wird lediglich noch der Drehdeckel 145 mit seinem Innengewinde in dem zylindrischen Vorsprung 146 mit dem Außengewinde in dem Gewindeelement 132 gekoppelt und dann durch den Schnappverschluss 144 auf dem Behälter 102 aufgesetzt.

Bezüglich der Figuren 1A-H sei darauf hingewiesen, dass beispielsweise die Membran 152 nicht ziehhärmonika-artig gebildet sein muss. Mit der Ausnutzung der Dehnungsfähigkeit des Membranmaterials könnte die Membran 152 in ihrem entspannten Zustand auch lediglich eine im wesentlichen zylindrische Form besitzen und sich bei Bewegung des Kolben-Stopfens 120 nach unten lediglich in Längsrichtung ausdehnen. Auch eine sich nicht dehnende Membran 152 wäre denkbar, die in einem entspannten Zustand eine im wesentlichen zylindrische Form mit einer Länge aufweist, die ausreicht, um in dem Zustand von Fig. 1A sich zwischen den beiden Dichtwulstbefestigungsorten zu erstrecken. Das Vorsehen der Ringfalten in der gezeigten ziehharmonika-artig gebildeten Membran bietet jedoch den Vorteil, dass bei dem Ausdehnen und Stauchen der Membran 152 das Zusammenfalten bzw. Ausfalten der Membran 152 in einer geordneten Art und Weise stattfindet, so dass die durch die Membran 152 auf den Kolben 116 wirkenden Kräfte besser prognostizierbar sind.

Ferner wird noch darauf hingewiesen, dass im vorhergehenden zwar nicht näher auf die Form des Verschlussstopfens 126 eingegangen worden ist, die Form des Verschlussstopfens 126 und der Öffnung 114 aber vorzugsweise derart aufeinander abgestimmt sind, dass in dem gequetschten Zustand nach Fig. 1A der Verschlussstopfen 126 wie beschrieben bündig mit der Innenoberfläche des Kanals 110 abschließt.

Schließlich sei darauf hingewiesen, dass die Belüftungsöffnung 156 mit dem Filter 158 nicht unbedingt vorgesehen sein müssen. Denn zunächst einmal ist das Volumen des Raumes 153 in dem Zustand in Fig. 1A gering, so dass das Volumen auch noch in dem Zustand in Fig. 1D in dem verbleibenden oberen Raum des Abschnitt 102a unterhalb der Verdrehsicherungsscheibe 137 unterbringbar ist. Und zusätzlich kann die Membran 153 beispielsweise aus einem derartigen Material gebildet sein, dass sie bei Normalbetriebstemperaturen den Raum 153 gegenüber dem Kolbenstopfenträger 118 keimdicht abschließt, und bei Änderung der Temperatur allerdings, wie z.B. bei Erhöhen der Temperatur, ein Be/Entlüften des Raumes 153 zu Sterilisationszwecken zulässt. Denkbar wäre ferner, dass der Dichtwulst 154 gerade eine so große Spannung bei Befestigung an dem Kolbenstopfenträger 118 aufweist, dass während der Sterilisation in Vakuumumgebung ein Gasaustausch möglich ist, während in dem Normalgebrauch jedoch die Keimdichtheit gewährleistet ist. In dem letztgenannten Fall könnte beispielsweise die Membran in der Nähe des Dichtwulstes 154 etwas steifer gebildet sein, so dass sie bei Ausbuchtung nach innen bei Anlegen des Vakuums beispielsweise um eine in dem Träger 118 vorgesehene Kante gedrückt wird, so dass der Dichtwulst 154 seine Passung in einer entsprechenden Nut in dem Träger 118 lockert.

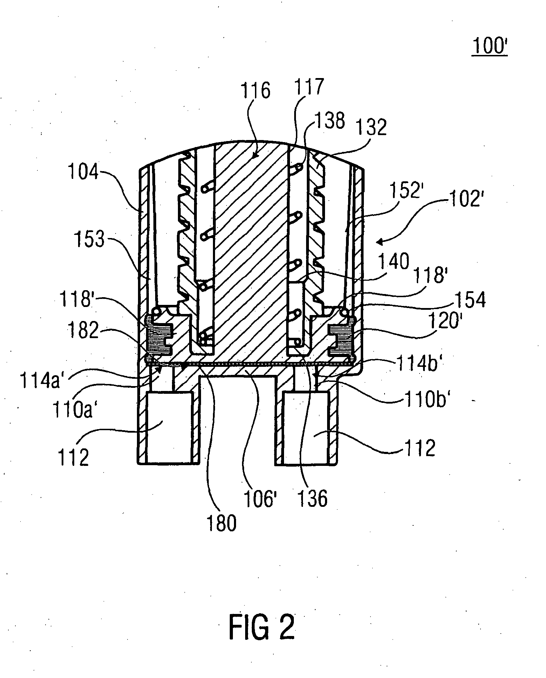

Im vorhergehenden wurde ein Ausführungsbeispiel für eine Fluidhandhabungsvorrichtung beschrieben, bei der das zur Fluidzwischenspeicherung vorgesehene Kolbenvolumen extern zu dem Kanal 110 vorgesehen war, aus dem das zwischenzuspeichernde Fluid durch die Fluidöffnung 114 entnommen werden sollte. In dem entleerten Zustand von Fig. 1A war dabei die Fluidöffnung 114 derart geschlossen worden, dass der Kanal 110 im wesentlichen eine Innenwand besaß, als ob die Fluidhandhabungsvorrichtung nicht da wäre. Im folgenden wird Bezug nehmend auf Fig. 2 ein dazu unterschiedliches Ausführungsbeispiel beschrieben, bei dem das Reservoirinnere als Teil des Durchflusskanals fungiert, d.h. unabhängig von der Stellung des Kolbens immer ein Spalt zwischen dem Behälterboden und dem Kolben verbleibt, über den zwei in dem Behälterboden vorgesehene Öffnungen kommunikativ miteinander verbunden sind, an die sich wieder der weitere Kanal anschließt.

Im folgenden wird das Ausführungsbeispiel von Fig. 2 näher beschrieben. Der Fluidhandhabungsbehälter von Fig. 2 ist allgemein mit dem Bezugszeichen 100' versehen und stimmt in vielen Teilen mit der Fluidhandhabungsvorrichtung 100 von Fig. 1A-H überein. Zur Vereinfachung der Beschreibung sind deshalb in Fig. 2 Bauteile, die zu denjenigen aus Fig. 1A-H identisch sind, mit gleichen Bezugszeichen versehen, wohingegen Elemente, die denjenigen aus Fig. 1A-H nur funktional entsprechen, aber etwas anders ausgebildet sind, ein ähnliches Bezugszeichen aufweisen, aber mit einem Apostroph versehen sind.

Die Fig. 2 zeigt den Fluidhandhabungsbehälter 100' in einer Kolbenstellung, der derjenigen von Fig. 1A entspricht. Der Kolben 116 befindet sich folglich in der unteren Stellung, niedergedrückt durch das untere Ende des Gewindeelementes 132, das unmittelbar auf den Kolbenstopfenträger bzw. den Kolbenstopfen 118' wirkt. Fig. 2 zeigt lediglich eine Detailansicht der Fluidhandhabungsvorrichtung 100', da diese Vorrichtung beispielsweise bezüglich des Drehdeckels, des Gewindeelementes 132, der Öffnung 156 mit Filter 158 und der Verdrehsicherungsscheibe 137 mit derjenigen von Fig. 1A-H übereinstimmt. Als ein anderes Beispiel für die Membran ist in Fig. 2 eine dehnfähige glatte im wesentlichen zylinderförmige Membran 152' gezeigt, die sich in dem Zustand von Fig. 2 in einer Situation maximaler Spannung befindet, eingeklemmt zwischen dem oberen Dichtwulst 150 (Fig. 1B) und einem unteren Dichtwulst 154, der wiederum unter Spannung in einer entsprechenden umlaufenden Nut in dem Kolbenstopfenträger 118' sitzt, um den Raum 153 keimdicht gegenüber dem mit außen kommunizierenden Gehäuseinneren abzuschließen. Der Gehäuseboden 106' weist im Unterschied zu dem Ausführungsbeispiel von Fig. 1A-H zwei Fluidöffnungen 114a' und 114b' auf, die in dem Ausführungsbeispiel von Fig. 2 exemplarisch näher am Rand des Bodens 106' angeordnet sind, wobei jedoch eine Anordnung möglich ist, bei der sich eine der Öffnungen 114a' und 114b' in der Mitte befindet. Zudem sind die Öffnungen 114a' und 114b' hier exemplarisch auf unterschiedlichen Radien bezüglich der Mitte des Bodens angeordnet, wobei beispielsweise die weiter von der Mitte entfernte Öffnung als Eingang fungiert bzw. zur proximalen Anordnung vorgesehen ist, während die andere als Ausgang fungiert bzw. zur distalen Anordnung vorgesehen ist. Die Anordnung beider Öffnungen auf gleichem Radius ist aber ebenfalls möglich. Denkbar ist auch eine Anordnung der Bohrungen wie in Fig. 1A, wobei die Mittelachsen der Anschlüsse 112 nicht parallel zur Mittelachse des Aufnahmebehälters 102', sondern zum Beispiel senkrecht dazu und zum Beispiel auf Höhe des Gehäusebodens 106' oder auch knapp darüber verlaufen können (nicht dargestellte Alternative).

Der Kolbenstopfenträger 118' ist nun im Unterschied zu dem Ausführungsbeispiel von Fig. 1A-H auf der dem Boden 106' zugewandten Seite zumindest in einem geschlossenen Bereich derselben, dem die beiden Fluidöffnungen 114a' und 114b' gegenüberliegen nicht mit dem elastischen Kolbenstopfenmaterial 120' versehen. Dies ist in Fig. 2 exemplarisch vielmehr lediglich an der der Innenwand 104 zugewandten Außenumfangskante an dem Kolbenstopfenträger 118' vorgesehen, um eine Ringdichtung zu bilden und eine fluiddichte Verdrängung durch den Kolbenstopfenträger 118' von Fluid aus dem Hubvolumen zu ermöglichen.

Das bedeutet, dass im Unterschied zu dem Ausführungsbeispiel von Fig. 1A-H auch kein elastisches Material an das Reservoirvolumen angrenzt, sondern das starre bzw. harte Material des Kolbens 116 selbst. Die ein vollständiges Entleeren ermöglichende Elastizitätseigenschaft ist bei dem Ausführuhgsbeisiel von Fig. 2 auch nicht notwendig, da bei diesem Ausführungsbeispiel ständig eine Fluidkommunikation zwischen den beiden Fluidöffnungen 114a' und 114b' aufrechterhalten werden soll. Um das zu gewährleisten, sind vorliegend exemplarisch an der dem Kolben 116 zugewandten Seite des Bodens 106' kleine vorstehende Abstandshalter 180 vorgesehen, die dafür sorgen, dass selbst dann, wenn sich der Kolben 116 in seiner tiefsten Stellung befindet, ein Spalt 182 zwischen Kolben 116 und Boden 106' erhalten bleibt, über welchen die Fluidöffnungen 114a' und 114b' fluidkommunikativ verbunden sind. Die Abstandshalter 118 könnten natürlich alternativ oder zusätzlich auf der dem Boden 106' zugewandten Seite des Kolbenstopfenträgers 118' vorgesehen sein.

Im Übrigen ist der Behälter 102' so geformt, dass sich an die Fluidöffnungen 114a' und 114b' jeweils noch ein Teil 110a' bzw. 110b' des Durchflusskanals anschließt, an deren den Öffnungen 114a, b' entgegengesetzten Enden jeweils ein Anschluss 112 vorgesehen ist.

Nachdem im vorhergehenden der Aufbau der Fluidhandhabungsvorrichtung 100' beschrieben worden ist, wird bezüglich der Funktionsweise derselben auf die vorhergehende Beschreibung bezüglich der Vorrichtung 100 verwiesen, zumindest soweit es die Kolbenbewegung und den Ans.aug- und den Ausstoßvorgang betrifft, mit dem Unterschied, dass hier zwei Fluidöffnungen 114a' und 114b' statt nur einer vorgsehen sind. Allerdings besteht bei der Vorrichtung 100' ständig eine Fluidkommunikation zwischen den beiden Anschlüssen 112 bzw. durch den Kanal 110a', 110b' und den Spalt 182. Auch bei dem Ausführungsbeispiel von Fig. 2 kann also Mischblut in dem Reservoirvolumen zwischengespeichert werden, wie z.B. in einer Anordnung, bei der die Vorrichtung 100' in eine Druckmesslinie geschaltet ist, so dass beispielsweise der Kanalabschnitt 110b' dem Patienten zugewandt ist und der Kanalabschnitt 110a' der Druckmessdose zugewandt ist. An einer patientennäher angeordneten Entnahmestelle könnte dann in dem zwischengespeicherten Zustand der Vorrichtung 100' eine Blutentnahme vorgenommen werden, woraufhin das zwischengespeicherte Blut wieder der Druckmesslinie zugeführt werden könnte.

Ein Vorteil der Anordnung von Fig. 2 gegenüber derjenigen von Fig. 1A-H besteht nur darin, dass in dem Fall von Fig. 2 an die Flüssigkeit innerhalb der Druckmesslinie und insbesondere an die Flüssigkeit innerhalb des Reservoirvolumens kein bzw. nur über eine geringe angrenzende Fläche hinweg elastisches Material 120' angrenzt, so dass eine Druckmessung an der Flüssigkeit nicht durch dieses elastische Material beeinflusst wird bzw., genauer ausgedrückt, Druckänderungen durch das elastische Material nicht gedämpft werden. Bei Fig. 2 grenzt vielmehr der starre Kolben 116 selbst an das Reservoirvolumen an.

dadurch, dass stets ein Spalt zwischen Kolben und Behälterboden erhalten bleibt, ist zwar kein vollständiges Auspressen der zwischengespeicherten Flüssigkeit, wie z.B. Mischblut, aus dem Reservoirvolumen möglich, aber das führt hier nicht zu Problemen in Bezug auf Koagulation oder dergleichen, da auch in dem Normalfall des heruntergedrückten Kolbens ein Fluss durch den Spalt erhalten bleibt, der das Reservoirinnere von Blutresten und dergleichen freispült.

Schließlich wird lediglich noch vorsichtshalber darauf hingewiesen, dass die Modifikationsmöglichkeiten, die Bezug nehmend auf das Ausführungsbeispiel von Fig. 1A-H explizit erwähnt wurden, natürlich auch auf das Ausführungsbeispiel von Fig. 2 übertragbar sind, solange sie nicht im Widerspruch zu der vorhergehenden Beschreibung der Fig. 2 stehen.

## Patentansprüche

1. Fluidhandhabungsvorrichtung (100) mit folgenden Merkmalen:
einem Aufnahmebehälter (102) mit einem darin verschiebbar angeordneten Kolben (116), derart, dass durch eine Verschiebung des Kolbens das Volumen eines Fluidaüfnahmereservoirs veränderbar ist;
einem Betätigungsmechanismus (142, 146, 132), der ausgebildet ist, um bei einer Betätigung desselben ein Mitnehmerlager (136) zu verschieben; und
einem Federmechanismus (138), der ausgebildet ist, um eine Kraftübertragung von dem Mitnehmerlager (136) auf den Kolben (116) zu bewirken, um ansprechend auf eine Verschiebung des Mitnehmerlagers (136) in eine erste Richtung eine solche Verschiebung des Kolbens (116) in dem Aufnahmebehälter (102) zu bewirken, dass ein Volumen des Fluidreservoirs (160) erhöht wird,
wobei der Betätigungsmechanismus (142, 146, 132) einen Drehkopf (142) mit einem Gewindemechanismus (146) aufweist, wobei das Mitnehmerlager (136) an einem Gewindeelement (132) angebracht ist, das ein Gewinde (134) aufweist, wobei der Gewindemechanismus (146) in das Gewinde (134) des Gewindeelementes (132) eingreift, so dass durch eine Drehung des Drehkopfes (142) die Verschiebung des Mitnehmerlagers (136) bewirkt werden kann.

2. Fluidhandhabungsvorrichtung (100) gemäß Anspruch 1, bei dem der Federmechanismus eine Schraubenfeder (138) oder eine Gasfeder ist.

3. Fluidhandhabungsvorrichtung (100) gemäß Anspruch 1 oder 2, bei der das Mitnehmerlager (136) und der Kolben derart ausgebildet sind, dass bei einer Verschiebung des Mitnehmerlagers (136) in einer zweiten Richtung das Mitnehmerlager (136) mit dem Kolben Eingriff nimmt, um eine solche Verschiebung des Kolbens zu bewirken, dass das Volumen des Fluidaufnahmereservoirs reduziert wird.

4. Fluidhandhabungsvorrichtung (100) gemäß einem der Ansprüche 1 bis 3, bei der der Kolben (116) eine Kolbenstange aufweist, die mit einer Kolbenscheibe (128) versehen ist, wobei der Federmechanismus (138) zwischen der Kolbenscheibe (128) und dem Mitnehmerlager (136) angeordnet ist.

5. Fluidhandhabungsvorrichtung (100) gemäß Anspruch 3 oder 4, bei der durch eine Verschiebung des Mitnehmerlagers in eine erste Richtung der Federmechanismus (138) zwischen dem Mitnehmerlager und der Kolbenstange gespannt wird.

6. Fluidhandhabungsvorrichtung (100) gemäß einem der Ansprüche 1 bis 5, bei dem das Gewinde (134) eine Gewindesteigung aufweist, derart, dass eine maximale Verschiebung des Mitnehmerlagers (136) durch eine Umdrehung des Drehkopfes (142) oder weniger bewirkt werden kann.

7. Fluidhandhabungsvorrichtung (100) gemäß einem der Ansprüche 1 bis 6, bei dem das Gewindeelement (132) eine Innenbohrung (130) aufweist, an deren Ende das Mitnehmerlager (136) gebildet, ist, wobei der Kolben (116) mit der Kolbenscheibe (128) die Innenbohrung (130) hineinragt.

8. Fluidhandhabungsvorrichtung (100) gemäß Anspruch 7, bei der die Innenbohrung (130) ausgebildet ist, um bei einer Verschiebung des Kolbens (116) als eine Führung der Kolbenscheibe (128) zu wirken, wobei der Federmechanismus (138) in der Innenbohrung (130) des Gewindeelementes (132) zwischen dem Mitnehmerlager (136) und der Kolbenscheibe (128) angeordnet ist.

9. Fluidhandhabungsvorrichtung (100) gemäß Anspruch 8, bei der die Innenbohrung (130) eine Stufe (140) aufweist, die ausgebildet ist, um einen minimalen Abstand von der Kolbenscheibe (128) und dem Mitnehmerlager (136) sicherzustellen und eine unmittelbare Kraftübertragung in dem Fall einer Bewegung des Gewindeelements 132 in die erste Richtung von dem Gewindeelement 132 auf den Kolben 116 zu ermöglichen.

10. Fluidhandhabungsvorrichtung (100) gemäß einem der vorhergehenden Ansprüche, wobei der Kolben (116) einen Kolbenstopfen (118,120) mit einem reversibel verformbaren Material (120) aufweist, wobei durch eine Bewegung des Kolbenstopfens (118,120) zu einem planaren Behälterboden Fluid aus der in dem planaren Behälterboden (106) angeordneten Fluidöffnung (114) treibbar ist, wobei der Kolbenstopfen in der dem planaren Behälterboden (106) zugewandeten Oberflächen eine Ausnehmung (122) aufweist, derart, dass, wenn der Kolbenstopfen gegen den planaren Behälterboden (106) gedrückt wird, bei zunehmenden Druck eine Berührungsgrenzlinie zwischen dem Kolbenstopfen (118,120) und dem Behälterboden (106) zu der Fluidöffnung (114) wandert.

11. Fluidhandhabungsvorrichtung 100' gemäß einem der Ansprüche 1 bis 9, bei dem der Aufnahmebehälter einen Behälterboden (106') mit zwei Fluidöffnungen (114a', 114b') aufweist, wobei an einer dem Boden (106') zugewandten Seite des Kolbens (116) und/oder auf einer dem Kolben (116) zugewandten Seite des Bodens (106') ein Abstandshalter (180) angeordnet ist, um unabhängig von einer Stellung des Kolbens (116) eine Fluidkommunikation zwischen den zwei Fluidöffnungen zu ermöglichen.

12. Fluidhandhabungsvorrich'tung gemäß einem der vorhergehenden Ansprüche, bei der der Kolben (116) mit einem Kolbenstopfen (118, 120) so in dem Aufnahmebehälter (102) angeordnet ist, dass das Fluidaufnahmereservoir (160) zwischen dem Kolbenstopfen (118, 120), einer Wand (104) des Aufnahmebehälters und einem Behälterboden (106) des Aufnahmebehälters, in dem eine Fluidöffnung (114) gebildet ist, gebildet ist, wobei die Fluidhandhabungsvorrichtung ferner eine Dichtvorrichtung (152) aufweist, die an dem Aufnahmebehälter (102) und dem Kolben (116) befestigt ist, um einen ersten Bereich (153) des Aufnahmebehälters benachbart zu einem Teil der Wand (104) des Aufnahmebehälters (102), über den der Kolbenstopfen des Kolbens verschiebbar ist, gasdicht von einem zweiten Bereich des Aufnahmebehälters abzuschirmen, der den Kolben umgibt.

13. Fluidhandhabungsvorrichtung (100) gemäß Anspruch 12, die ferner folgendes Merkmal aufweist:
eine Öffnung (156) in der Wand, die mit dem ersten Bereich (153) fluidisch verbunden und außerhalb des Teils der Wand angeordnet ist, über den der Kolbenstopfen des Kolbens verschiebbar ist, zur Be- und Entlüftung des ersten Bereichs (153).

14. Fluidhandhabungsvorrichtung (100) gemäß Anspruch 12 oder 13, wobei in der Öffnung (156) oder an der Öffnung (156) ein Filter (158) angeordnet ist, um bei einer Belüftung des ersten Bereichs (153) Keime an einem Eindringen in den ersten Bereich (153) zu hindern.

15. Fluidhandhabungsvorrichtung (100), gemäß Anspruch 14, bei dem der Filter (158) ausgebildet ist, um ein biologisches, infektiöses Material, insbesondere Bakterien, Viren und Keime, am Eindringen zu hindern.

16. Fluidhandhabungsvorrichtung (100), gemäß einem der Ansprüche 12 bis 15, bei der die Dichtvorrichtung (152) ausgebildet ist, um sich bei einer Bewegung des Kolbens (116) zusammen- bzw. auseinanderzufalten.

## Claims

1. Fluid handling device (100) comprising:
a receiving container (102) with a piston (116) arranged therein in a displaceable manner, such that the volume of a fluid receiving reservoir may be changed by a displacement of the piston;
an actuation mechanism (142, 146, 132) configured to displace a carrier bearing (136) upon actuation of the former; and
a spring mechanism (138) configured to transfer a force from the carrier bearing (136) to the piston (116) so as to effect, in response to displacement of the carrier bearing (136) in a first direction, a displacement of the piston (116) within the receiving container (102) such that a volume of the fluid reservoir (160) is increased,
the actuation mechanism (142, 146, 132) comprising a rotary knob (142) with a thread mechanism (146), the carrier bearing (136) being mounted on a thread element (132) comprising a thread (134), the thread mechanism (146) engaging with the thread (134) of the thread element (132) so that the displacement of the carrier bearing (136) may be effected by a rotation of the rotary knob (142).

2. Fluid handling device (100) as claimed in claim 1, wherein the spring mechanism is a coil spring (138) or a gas spring.

3. Fluid handling device (100) as claimed in claims 1 or 2, wherein the carrier bearing (136) and the piston are configured such that, upon a displacement of the carrier bearing (136) in a second direction, the carrier bearing (136) engages with the piston so as to effect a displacement of the piston such that the volume of the fluid receiving reservoir is reduced.

4. Fluid handling device (100) as claimed in any of claims 1 to 3, wherein the piston (116) comprises a piston rod provided with a piston disc (128), the spring mechanism (138) being arranged between the piston disc (128) and the carrier bearing (136).

5. Fluid handling device (100) as claimed in claims 3 or 4, wherein the spring mechanism (138) between the carrier bearing and the piston rod is tensioned due to a displacement of the carrier bearing in a first direction.

6. Fluid handling device (100) as claimed in any of claims 1 to 5, wherein the thread (134) comprises a thread pitch such that a maximum displacement of the carrier bearing (136) can be effected by one revolution of the rotary knob (142) or less.

7. Fluid handling device (100) as claimed in any of claims 1 to 6, wherein the thread element (132) comprises an internal bore (130) at the end of which the carrier bearing (136) is formed, the piston (116) with the piston disc (128) projecting into the internal bore (130).

8. Fluid handling device (100) as claimed in claim 7, wherein the internal bore (130) is configured to act as a guide of the piston disc (128) upon displacement of the piston (116), the spring mechanism (138) being arranged, within the internal bore (130) of the thread element (132), between the carrier bearing (136) and the piston disc (128).

9. Fluid handling device (100) as claimed in claim 8, wherein the internal bore (130) comprises a stage (140) configured to ensure a minimum distance from the piston disc (128) and from the carrier bearing (136) and to enable direct transfer of forces from the thread element 132 to the piston 116 in the event of a movement of the thread element 132 in the first direction.

10. Fluid handling device (100) as claimed in any of the previous claims, wherein the piston (116) comprises a piston plug (118, 120) having a reversibly deformable material (120), it being possible for fluid to be driven out of the fluid opening (114) arranged in a planar container bottom (106) by a movement of the piston plug (118, 122) to said planar container bottom, the piston plug comprising a recess (122) in that surface which faces the planar container bottom (106), such that when the piston plug is pressed against the planar container bottom (106), a contact boundary line between the piston plug (118, 120) and the container bottom (106) migrates toward the fluid opening (114) as the pressure increases.

11. Fluid handling device 100' as claimed in any of claims 1 to 9, wherein the receiving container comprises a container bottom (106') with two fluid openings (114', 114b'), a spacer (180) being arranged on a side of the piston (116) which faces the bottom (106') and/or on a side of the bottom (106') which faces the piston (116) so as enable fluidic communication between the two fluid openings independently of a position of the piston (116).

12. Fluid handling device as claimed in any of the previous claims, wherein the piston (116) with a piston plug (118, 120) is arranged within the receiving container (102) in such a manner that the fluid receiving reservoir (160) is formed between the piston plug (118, 120), a wall (104) of the receiving container and a container bottom (106) of the receiving container which has a fluid opening (114) formed therein, the fluid handling device further comprising a sealing device (152) attached to the receiving container (102) and to the piston (116) so as to shield off in a gas-tight manner a first area (153) of the receiving container adjacent to a portion of the wall (104) of the receiving container (102) across which the piston plug of the piston may be displaced, from a second area of the receiving container which surrounds the piston.

13. Fluid handling device (100) as claimed in claim 12, further comprising:
an opening (156) in the wall which is in fluidic communication with the first area (153) and is arranged externally to that portion of the wall across which the piston plug of the piston may be displaced, for the purpose of aerating and deaerating the first area (153).

14. Fluid handling device (100) as claimed in claims 12 or 13, a filter (158) being arranged in the opening (156) or at the opening (156) so as to prevent germs from entering the first area (153) during aeration of the first area (153).

15. Fluid handling device (100) as claimed in claim 14, wherein the filter (158) is configured to prevent biological, infectious material, in particular bacteria, viruses and germs, from entering.

16. Fluid handling device (100) as claimed in any of claims 12 to 15, wherein the sealing device (152) is configured to fold up and unfold upon a movement of the piston (116).

## Revendications

1. Dispositif de manipulation de fluide (100), avec les caractéristiques suivantes:
un conteneur de réception (102) avec un piston (116) y disposé de manière déplaçable, de sorte que par un déplacement du piston peut être modifié le volume d'un réservoir de réception de fluide;
un mécanisme d'actionnement (142, 146, 132) conçu pour déplacer, lors d'un actionnement de celui-ci, un palier d'entraînement (136); et
un mécanisme de ressort (138) conçu pour provoquer une transmission de force du palier d'entraînement (136) au piston (116), pour provoquer, en réponse à un déplacement du palier d'entraînement (136) dans une première direction, un déplacement du piston (116) dans le conteneur de réception (102) de sorte que soit augmenté un volume du réservoir de fluide (160),
dans lequel le mécanisme d'actionnement (142, 146, 132) présente une tête rotative (142) avec un mécanisme fileté (146), dans lequel le palier d'entraînement (136) est placé sur un élément fileté (132) qui présente un filetage (134), le mécanisme fileté (146) s'engageant dans le filetage (134) de l'élément fileté (132), de sorte que par une rotation de la tête rotative (142) puisse être provoqué le déplacement du palier d'entraînement (136).

2. Dispositif de manipulation de fluide (100) selon la revendication 1, dans lequel ledit mécanisme de ressort est un ressort hélicoïdal (138) ou un ressort à gaz.

3. Dispositif de manipulation de fluide (100) selon la revendication 1 ou 2, dans lequel le palier d'entraînement (136) et le piston sont conçus de sorte que, lors d'un déplacement du palier d'entraînement (136) dans une seconde direction, le palier d'entraînement (136) vienne en prise avec le piston, pour provoquer un déplacement du piston de sorte que soit réduit le volume du réservoir de réception de fluide.

4. Dispositif de manipulation de fluide (100) selon l'une des revendications 1 à 3, dans lequel le piston (116) présente une tige de piston qui est pourvue d'un disque de piston (128), le mécanisme de ressort (138) étant disposé entre le disque de piston (128) et le palier d'entraînement (136).

5. Dispositif de manipulation de fluide (100) selon la revendication 3 ou 4, dans lequel, par un déplacement du palier d'entraînement dans une première direction, le mécanisme de ressort est tendu (138) entre le palier d'entraînement et la tige de piston.

6. Dispositif de manipulation de fluide (100) selon l'une des revendications 1 à 5, dans lequel le filet (134) présente un pas de vis de sorte qu'un déplacement maximum du palier d'entraînement (136) puisse être provoqué par une rotation de la tête rotative (142) ou moins.

7. Dispositif de manipulation de fluide (100) selon l'une des revendications 1 à 6, dans lequel l'élément fileté (132) présente un alésage intérieur (130) à l'extrémité duquel est formé le palier d'entraînement (136), le piston (116) avec le disque de piston (128) pénétrant à l'intérieur de l'alésage (130).

8. Dispositif de manipulation de fluide (100) selon la revendication 7, dans lequel l'alésage intérieur (130) est conçu pour agir, lors d'un déplacement du piston (116), comme guide du disque de piston (128), le mécanisme de ressort (138) étant disposé dans l'alésage intérieur (130) de l'élément fileté (132), entre le palier d'entraînement (136) et le disque de piston (128).

9. Dispositif de manipulation de fluide (100) selon la revendication 8, dans lequel l'alésage intérieur (130) présente un étage (140) qui est conçu pour assurer une distance minimale par rapport au disque de piston (128) et au palier d'entraînement (136) et permettre, en cas de déplacement de l'élément fileté 132 dans la première direction, une transmission directe de force de l'élément fileté 132 au piston 116.

10. Dispositif de manipulation de fluide (100) selon l'une des revendications précédentes, dans lequel le piston (116) présente un bouchon de piston (118, 120) en un matériau déformable de manière réversible (120), du fluide pouvant être sorti, par un déplacement du bouchon de piston (118, 120) vers un fond de conteneur plan, d'une orifice à fluide (114) disposé dans le fond du conteneur plan (106), le bouchon de piston présentant, dans la surface orientée vers le fond du conteneur plan (106), un évidement (122), de sorte que, lorsque le bouchon de piston est poussé contre le fond du conteneur plan (106), une limite de contact entre le bouchon de piston (118, 120) et le fond du conteneur (106) avec ladite orifice à fluide (114) varie au fur et à mesure que la pression augmente.

11. Dispositif de manipulation de fluide 100' selon l'une des revendications 1 à 9, dans lequel le conteneur de réception présente un fond de conteneur (106') avec deux orifices à fluide (114a', 114b'), sur un côté du piston (116) orienté vers le fond (106'), et/ou sur un côté du fond (106') orienté vers le piston (116) étant disposée une entretoise (180) pour permettre, indépendamment d'une position du piston (116), une communication en fluide entre les deux orifices à fluide.

12. Dispositif de manipulation de fluide selon l'une des revendications précédentes, dans lequel le piston (116) avec un bouchon de piston (118, 120) est disposé dans le conteneur de réception (102) de sorte que le réservoir de réception de fluide (160) soit formé entre le bouchon de piston (118, 120), une paroi (104) du conteneur de réception et un fond de conteneur (106) du conteneur de réception dans lequel est formé un orifice à fluide (114), le dispositif de manipulation de fluide présentant par ailleurs un dispositif d'étanchéité (152) qui est fixé au conteneur de réception (102) et au piston (116) pour blinder de manière étanche au gaz une première zone (153) du conteneur de réception adjacente à une partie de la paroi (104) du conteneur de réception (102) sur laquelle est déplaçable le bouchon du piston d'une deuxième zone du conteneur de réception entourant le piston.

13. Dispositif de manipulation de fluide (100) selon la revendication 12, présentant par ailleurs:
un orifice (156) dans la paroi, qui est en communication de fluide avec la première zone (153) et disposé à l'extérieur de la partie de la paroi sur laquelle est déplaçable le bouchon du piston, pour ventiler et purger d'air la première zone (153).

14. Dispositif de manipulation de fluide (100) selon la revendication 12 ou 13, dans lequel dans l'orifice (156) ou à l'orifice (156) est disposé un filtre (158) pour empêcher que, lors d'une aération de la première zone (153), des germes ne pénètrent dans la première zone (153).

15. Dispositif de manipulation de fluide (100) selon la revendication 14, dans lequel le filtre (158) est conçu pour empêcher que ne pénètrent une matière biologique, une matière infectieuse, en particulier des bactéries, des virus et des germes.

16. Dispositif de manipulation de fluide (100) selon l'une des revendications 12 à 15, dans lequel le dispositif d'étanchéité (152) est conçu de manière à se replier ou se déplier lors d'un mouvement du piston (116).
